# EUROPEAN PATENT APPLICATION

(11) **EP 1 642 574 A2**
(43) Date of publication of application: **05.04.2006**
(21) Application number: 05013726.4
(22) Date of filing: 22.04.2003
(51) Int. Cl.: A61K 31/135, A61K 31/137, A61K 31/7008, A61P 29/00

(54) **Combination of a beta-2 adrenoceptor agonist and an aminosugar and their use for the treatment of immunomodulatory disorders**

(30) Priority: 19.04.2002 DK 200200586; 19.04.2002 US 373615 P
(62) Divisional of application: 03752710.8
(71) Applicant: Astion Development A/S, 2100 Copenhagen O (DK)
(72) Inventor: Weidner, Morten Sloth, 2830 Virum (DK)
(74) Representative: Plougmann & Vingtoft A/S

(57) **Abstract**

The invention relates to combinations of an aminosugar and a beta-2 adrenoceptor agonist, such as salbutamol, for the treatment of diseases associated with hypersensitivity and inflammation, in particular hypersensitivity skin diseases. The aminosugar is preferably a monosaccharide derivative.

## Description

### FIELD OF THE INVENTION

The present invention relates to the combination of a beta-2 adrenoceptor agonist and an aminosugar suitably formulated in the form of a chemical complex and/or a pharmaceutical composition for the suppression and treatment of hypersensitivity and inflammatory reactions in mammals.

### BACKGROUND OF THE INVENTION

A number of drug classes are available for the treatment of hypersensitivity and inflammatory reactions. Among these, the corticosteroids are some of the most widely and effective drugs used. Corticosteroids primarily exert their pharmacological action by non-selectively inhibiting the function and proliferation of different classes of immune cells resulting in suppression of hypersensitivity and inflammatory reactions. Unfortunately, the corticosteroids are associated with a number of serious side effects, e.g. immunosuppression, osteoporosis and skin atrophy.

Non-steroidal anti-inflammatory drugs are another class of drugs extensively used in the treatment of hypersensitivity and inflammatory reactions. Also this class of drugs is associated with serious side effects, in particular upon long-term use.

Hypersensitivity is defined as a state of altered reactivity in which the body reacts with an exaggerated immune response to a substance (antigen).

Hypersensitivity reactions underlie a large number of diseases. Among these, allergic and autoimmune conditions are of great importance. A classification of hypersensitivity diseases is given in the textbook Clinical Medicine (Kumar, P. and Clark, M.: "Clinical Medicine", 3rd edition, p. 147-150, 1994, Bailliere Tindall, London). Hypersensitivity may be classified as type I hypersensitivity reactions (IgE mediated allergic reactions) which is known to play a significant role include asthma, eczema (atopic dermatitis), urticaria, allergic rhinitis and anaphytaxis. Type II hypersensitivity reactions are caused by cell surface or tissue bound antibodies (IgG and IgM) and play a significant role in the pathogenesis of myasthenia gravis, Good-pasture's syndrome and Addisonian pernicious anaemia. Type III hypersensitivity reactions (immune complex) are caused by autoantigens or exogenous antigens, such as certain bacteria, fungi and parasites.

Diseases in which type III hypersensitivity reactions play a significant role include lupus erythematosus, rheumatoid arthritis and glomerulonephritis. Type IV hypersensitivity reactions (delayed) are caused by cell or tissue bound antigens. This type of hypersensitivity plays a significant role in a number of conditions, e.g. graft-versus-host disease, leprosy, contact dermatitis and reactions due to insect bites.

In addition cancer may be regarded as a condition associated with hypersensitivity reactions. Cancer is caused by an uncontrolled proliferation of cells that express varying degrees of fidelity to their precursors. These cancer cells form a malignant tumour that enlarges and may spread to adjacent tissues or through blood and lymph systems to other parts of the body. There are numerous forms of cancer of varying severity. For most types of cancer there is no effective treatment today.

Generally, the treatment of hypersensitivity and inflammatory diseases, including cancer, requires long-term administration. Thus, there is a need for therapeutic agents for the treatment of hypersensitivity and inflammatory reactions, including cancer, in particular agents that have a better safety profile than presently available drugs.

Aminosugars are generally recognised as having beneficial effect on inflammatory reactions. Aminosugars are the building blocks for the *in vivo* generation of glycosaminoglycans, formerly known as mucopolysaccharides. Glycosaminoglycans are constituents in various tissues in numerous mammals, both vertebrates and invertebrates and as such not likely to be associated with adverse reactions upon administration to mammals. Important examples of glycosaminoglycans are chondroitin sulfates, keratan sulfates in connective tissue, dermatan sulfates in skin tissue and hyaluronic acid in skin tissue and synovial joint fluid.

Administration of aminosugars or glycosaminoglycans in high (pharmacological) doses to individuals suffering from osteoarthritis has resulted in some relief of symptoms and nowadays the use of aminosugars as chondroprotective agents is widely recognised *(Gaby AR, Natural treatments for osteoarthritis, Alternative medicine review, volume 4, No 5, 1999, pages 330-334).* For example, the use of aminosugars and glycosaminoglycans for reducing inflammation is mentioned in WO 98/48816. US 6.046,179 relates to the treatment of inflammatory bowel diseases by colonic administration of N-acetylglucosamine.

Sympathomimetics are drugs that partially or completely mimic the actions of noradrenaline or adrenaline. They act either directly on alpha- and/or beta-adrenoceptors or indirectly on the presynaptic terminals usually by causing the release of noradrenaline.

The effects of adrenoceptor stimulation are various. Beta-2 adrenoceptor agonists are a class of drugs known to provide bronchodilation and are widely used in the treatment of asthma. WO 95/19336 relates to phenyl ethanol amine ethers for use as a beta-2 adrenoceptor agonists in bronchitis, allergic bronchitis and astma bronchiale.

EP 069042 relates to drug compositions comprising a mucopolysaccharide and a drug which is scarcely soluble in water but soluble in a water-miscible organic solvent, such as salbutamol. The drug is present as fine crystals or fine particles attached on or between the particles of a mucopolysaccharide.

### SUMMARY OF THE INVENTION

It has been found by the present investigator that a combination of a beta-2 adrenoceptor agonist and an aminosugar significantly suppresses hypersensitivity and inflammatory reactions.

Contrarily to existing therapeutic agents, such as corticosteroids or non-steroidal anti-inflammatory drugs, the chemical complexes and compositions according to the present invention have the advantage of not being likely to be associated with any serious side effects, as all of their components are known to living organisms and are acknowledged reported as non-toxic and well-tolerated by the organism. The present inventor puts forward the hypothesis that the very beneficial therapeutic index exhibited by the complex and compositions comprising said complex according to the invention is superior to the use of the individual constituents of the complex, and this is due to synergistic effects and a lower toxic load on the organism.

Such a combination is advantageously provided in the form of a chemical complex comprising a beta-2 adrenoceptor agonist and an aminosugar. Obviously, the combination may also be provided in the form of a pharmaceutical composition, a dietary supplement or a cosmetic. As was further recognised by the present inventor, the aminosugar according to the present invention may be an aminosugar derivative of monosaccharides, oligosaccarides as well as of polysaccharides. However, the aminosugar may advantageously have a molecular weight of less than 5000.

Thus, the present inventor has recognised the therapeutic activity of a combination of beta-2 adrenoceptor agonist and an aminosugar, for which reason the said combination may be regarded as an active therapeutic agent.

Accordingly, the present invention provides a chemical complex or a pharmaceutical composition comprising:
i) a beta-2 adrenoceptor agonist; and
ii) an aminosugar; and optionally
iii) a pharmaceutically acceptable carrier or carrier.
   The chemical complexes and pharmaceutical compositions according to the invention may in general be utilised in the treatment of diseases associated with hypersensitivity and inflammatory reactions. In general the combination may be utilised in i) immunomodulation, and in more specific terms they may be utilised in ii) the treatment or prevention of hypersensitivity diseases such as atopic eczema, contact dermatitis, seborrhoeic eczema and/or psoriasis; ii) the treatment or prevention of IgE mediated allergic reactions and conditions such as of asthma, allergic rhinitis, and/or anaphylaxis;
iv) the treatment or prevention of autoimmune disorders such as of diabetes, Crohn's disease, ulcerative colitis, rheumatoid arthritis, gout or osteoarthritis; v) the alleviation of pain; vi) the treatment or prevention of cancer.

An important aspect of the invention relates to the use of a combination of a beta-2 adrenoceptor agonist and an aminosugar for the preparation of a product for the treatment of diseases i) to vi) as mentioned above.

Still further aspects relate independently to a method for treating diseases i) to vi) as mentioned above in a mammal, such as a human, comprising the administration of a combination of a beta-2 adrenoceptor agonist and an aminosugar, pharmaceutically acceptable salts thereof, or a complex comprising said combination or said salts to said mammal.

Moreover, a still further aspect of the invention relates to a process for the preparation of a complex comprising i) a beta-2 adrenoceptor agonist; and ii) an aminosugar, comprising the steps of:
i) dissolving said beta-2 adrenoceptor and said aminosugar in a volatile solvent or a mixture of volatile solvents; and
ii) removing said suitable solvent so as to obtain a moisture content of at the most 5% w/w.

### DETAILED DESCRIPTION OF THE INVENTION

The present inventor provides data herein indicating that a combination of a beta-2 adrenoceptor agonist and an aminosugar significantly reduces the inflammation in the arachidonic acid ear inflammation test in mice. This reduction of inflammation was better for the combination than for each of the individual compounds and also far better than that obtained by a commonly used steroid.

It is hypothesised by the present inventor that the very advantageous therapeutic index of the combination of a beta-2 adrenoceptor agonist and an aminosugar in comparison to each of the singular components is due to synergistic effects between the components of the compositions. Advantageously, this allows for the utility of lower dosages, while yet providing a surprisingly good therapeutic effect.

The invention is based, at least in part, on the combined activity of an aminosugar and a beta-2 adrenoceptor agonist in comparison to either component. This combined activity allows for the use of beta-2 adrenoceptor agonists that are previously not used as therapeutic agents because they were too toxic in therapeutically relevant doses or because high doses were required in order to achieve said effect.

According to the invention, the combination of a beta-2 adrenoceptor agonist and an aminosugar may be provided in the form of a chemical complex; in the form of a composition comprising said complex and optionally pharmaceutically acceptable excipient(s); or in the form of a pharmaceutical composition comprising the combination of beta-2 adrenoceptor agonist and an aminosugar.

Without being limited to a particular theory, advantageously, said combination is provided in the form of a chemical complex for purposes of achieving a homogeneous mixture of the two agents, which may positively affect the resulting therapeutic effect.

Such chemical complexes are novel and provide a surprisingly effective anti-hypersensitivity and anti-inflammatory effect with a surprisingly good safety profile. Thus the chemical complexes or compositions of the invention are virtually non-toxic at active doses and yet very therapeutically effective.

The chemical complexes or compositions of the invention provide pharmacological effects upon administration to the living organism such as immunomodulation, suppression of hypersensitivity reactions, suppression of IgE mediated allergic reactions, suppression of autoimmune reactions, reduction of pain, and suppression of cancer.

Accordingly, the present invention relates to a chemical complex comprising:
i) a beta-2 adrenoceptor agonist; and
ii) an aminosugar.

The term "chemical complex" is intended to include the definition defined by IUPAC that read as follows:
*"A molecular entity formed by loose association involving two or more component molecular entities (ionic or uncharged), or the corresponding chemical species. The bonding between the components is normally weaker than in a covalent bond. "(IUPAC Compendium of Chemical Terminology 2nd Edition (1997))*

Thus, the term "chemical complex" is intended to mean any combination of the components provided that the molecules of each of the components are mixed and loosely associated with each other. The term "chemical complex" is not intended necessarily to implie an ionic or otherwise association between the components. It does not either include covalent bonding between the components of the complex. Moreover, the term "chemical complex" does not encompass combinations wherein one or both of the components are in the form of partides. However, a chemical complex of the invention may not be 100 % pure in that some of the components may be present in the form of particles. That is to say that preferably less than 10% of each of the components are in the form of particles in a chemical complex. More preferably less than 5%, less than 2.5% or less than 1% is in particulate matter. Thus, a composition or a chemical complex according to the invention may comprise less than 10% of one of the components in the form of particulate matter.

The complexes of the invention may be prepared according to a number of different methods, which are obvious to a person skilled in the art. The following procedures are non-limiting examples of such methods:
The components of the complex, dosed in appropriate amounts to give the correct molar ratio between the components, are dissolved, dispersed, or suspended in an appropriate solvent, for example water, an organic solvent or mixtures thereof. Non-limiting examples of suitable organic solvents are ethanol, methanol, iso-propyl alcohol, acetone, hexane, ethylacetate or mixtures thereof.
The solvent is then removed by a technique suitable for the complex, for example but not limited to evaporation, *in vacou* evaporation, spray drying, freeze-drying, fluid bed drying or spin flash drying. Alternatively, the complex may be obtained by precipitation and subsequent centrifugation or filtering.

In the present context, the term "aminosugar" is intended to mean one or more amino derivatives of a monosaccharide (aldoses and ketoses) and its corresponding sugar alcohols (alditols) such as trioses, tetroses, pentoses, hexoses, heptoses and octoses. The aldose, ketose, or aiditol has one or more hydroxy groups replaced by any amino group at any position, including the anomeric position. An aminosugar is thus a deoxyamino derivative of an aldose, ketose, or alditol. The term is also intended to mean polyamino sugars, wherein more than one hydroxy group has been replaced by an amino group (e.g. dideoxydiamino-, trideoxytriamino-derivatives).

Moreover, the term "aminosugar" is also intended to mean amino derivatives of di-, oligo-and poly-saccharides comprising at least one of said monosaccharides. Consequently, in the case of di-, oligo- and poly-saccharides, the amino group may be the position of glycosidation. Suitably, in di-, oligo- and poly-saccharides, the amino group may not be the position of glycosidation.

An amino group of an aminosugar may be alkylated, arylated or acylated or, alternatively, present as its free amine form (NH₂). Similarly, the hydroxyl groups may be optionally protected or derivatised such as alkylated, arylated or acylated or, altematively, present in its free hydroxyl form.

The amine of the amino sugar may exist as its quaternary ammonium salt using organic or mineral acids, as is known to the person skilled in the art. Furthermore, other functional groups on the aminosugar may be in the form of a salt. Similarly, prodrug derivatives of the aminosugar are anticipated by the present inventor. The prodrug form may be the result of the derivatisation of the amino group or another functional group present on the aminosugar, as is known to the person skilled in the art.

Furthermore, an aminosugar may have one or more hydroxy groups replaced by any amino group at any position and a further one or more hydroxy groups replaced by a hydrogen (a deoxy sugar), a thiol (a thiosugar), a halogen (a deoxyhalo sugar), an anhydrosugar (a sugar preparable via an intramolecular displacement with a hydroxyl to form an oxirane or oxetane), a carbonyl group.

Furthermore, the term aminosugar is denoted to mean aminosugars as described *supra* but optionally substituted.

The term "optionally substituted" is intended to mean the substitution of one or more hydrogen atoms, which is substituted with another atom, chemical group or entity, termed substituents. Illustrative examples of substituents include carboxyl, formyl, amino, hydroxyl, halogen, nitro, sulphono, sulphanyl, C₁₋₆-alkyl, aryl, aryloxy, aryloxycarbonyl, arylcarbonyl, heteroaryl, amino, mono- and di(C₁₋₆-alkyl)amino; carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, cyano, guanidino, carbamido, C₁₋₆-alkanoyloxy, C₁₋₆-alkylsulphonyloxy, dihalogen-C₁₋₆-alkyl, trihalogen-C₁₋₆-alkyl, C₁₋₆-alkoxyl, oxo, C₁₋₆-carboxyl, C₁₋₆-alkoxycarbonyl, C₁₋₆-alkylcarbonyl, where aryl and heteroaryl representing substituents may be substituted 1-5 times with C_{1- 6}-alkyl, C₁₋₆-alkoxy, nitro, cyano, hydroxy, amino or halogen. In general, the above substituents may be susceptible to further optional substitution.

The term "halogen" includes fluorine, chlorine, bromine and iodine.

In a particularly suitable embodiment of the invention, the aminosugar is sulphated or phosphorylated at the anomeric, 2-, 3-, 4-, or 6- position, typically at the 2-, 3-, or 4-position. In another suitable embodiment of the invention the aminosugar is N-acetylated.

Furthermore, a combination of suitable embodiments include the aminosugar sulphated or phosphorylated as well as in its salt form having Na⁺; K⁺; Mg⁺⁺; Ca⁺⁺; or NH₄⁺ as counter ions.

Particularly suitable aminosugars according to the invention are amino derivates of monosaccharides selected from the group consisting of glucosamine, galactosamine and mannosamine, derivatives and salts thereof. Typically, the amino derivates of monosaccharides may be in the form of salts, such as the sulfate salt and hydrochloride salts, or N-acetylated, e.g. glucosamine sulfate, glucosamine hydrochloride, N-acetylglucosamine, galactosamine sulfate, galactosamine hydrochloride, N-acetylgalactosamine, mannosamine sulfate, mannosamine hydrochloride, N-acetylmannosamine, as well as other aminosugars known to the person skilled In the art.

In suitable embodiments the aminosugar is di-, oligo-, and poly-saccharides comprising at least one or more of the mentioned amino derivates of monosaccharides. In the embodiment wherein the aminosugar is an oligo- or polysaccharide, said oligo- or polysaccharide preferably contain monomeric sugars including D-glucuronic acid, L-iduronic acid, D-galacturonic acid, D-galactose, and fucose, each of which may be optionally sulfonated or O-substituted with a protective group known to the person skilled in the art.

In a suitable embodiment of the invention, the chemical complex and the composition comprises more than one aminosugar.

Preferably, the aminosugar is an amino derivate of a monosaccharide as mentioned *supra.* In the embodiment wherein the aminosugar is oligo- and poly-saccharides the molecular weight is preferably less than 5000 Daltons, preferably less than 4000 Daltons, more preferably less than 3000 Daltons

The aminosugar component of the invention may comprise natural, synthetic or semisynthetic aminosugars and may have been chemically modified, while still retaining their function. Such chemical modifications include but are not limited to esterification, sulfation, polysulfation, acetylation and methylation.

As stated, the invention relates to the combination of an aminosugar with a beta-2 adrenoceptor agonist. The term "beta-2 adrenoceptor agonist" is intended to mean any component with the ability to stimulate a beta-2 adrenoceptor or parts thereof. The agonistic activity of a compound towards beta-2 adrenoceptor may be investigated by methods known to the person skilled in the art, eventually using salmeterol as reference. Preferably, the beta-2 adrenoceptor agonist may be any that possess at least 10% of the activity of salmeterol in a suitable test for beta-2 adrenoceptor agonism. Preferably, the beta-2 adrenoceptor agonist has at least 20%, more preferably at least 40% such as at least 50%, 60%, 75%, 80%, 85%, 90% of the activity of salmeterol in a suitable test for beta-2 adrenoceptor agonism.

The beta-2 adrenoceptor agonist, for illustrative purposes, may be selected from the group consisting of bambuterol, bitolterol, carbuterol, clenbuterol, clorprenaline, dioxethedrine, dopexamine, ephedrine, epinephrine, etafedrine, ethylnorepinephrine, fenoterol, formoterol, hexoprenaline, isoetarine, isoproterenol, mabuterol, metaproterenol, methoxyphenamine, pirbuterol, procaterol, protokylol, reproterol, rimiterol, ritodrine, salbutamol (albuterol), salmeterol, soterenol, terbutaline, tretoquinol, tulobuterol, derivatives, salts and enantiomeres thereof.

In interesting embodiments the beta-2 adrenoceptor agonist is terbutaline sulfate, salbutamol sulfate or formoterol fumarate dihydrate.

According to the invention the beta-2 adrenoceptor agonist may preferably be in the form of the most effective single enantiomer or optimal mixtures of enantiomers as known to a person skilled in the art.

As stated the combination of the two agents provides a surprisingly effective therapeutic agent for suppression of hypersensitivity and inflammatory reactions. The proper therapeutic efficacy may, in part, be adjusted by providing the two agents in suitable molar ratios or mass ratios.

The molar ratio between the beta-2 adrenoceptor agonist and the aminosugar may be about 1:10000 to 10000:1, preferably about 1:1000 to 1000:1, such as about 1:500 to 500:1, such as 1:100 to 100:1, about 1:50 to 50:1, or about 1:40 to 40:1, also about 1:30 to 30:1, such as about 1:25 to 25:1, about 1:20 to 20:1, about 1:18 to 18:1, about 1:16 to 16:1, about 1:14 to 14:1, or about 1:12 to 1:12, also about 1:10 to 10:1, such as about 1:9 to 9:1, about 1:8 to 8:1, about 1:7 to 7:1, about 1:6 to 6:1, also from 1:5 to 5:1, such as from 1:4 to 4:1, e.g. from 1:3 to 3:1, such as from 1:2 to 2:1.

Alternatively defined, the ratio between the beta-2 adrenoceptor agonist and the aminosugar may be expressed as a mass ratio. The mass ratio between the beta-2 adrenoceptor agonist and the aminosugar may be about 1:10000 to 10000:1, preferably about 1:1000 to 1000:1, such as about 1:500 to 500:1, such as 1:100 to 100:1, about 1:50 to 50:1, or about 1:40 to 40:1, also about 1:30 to 30:1, such as about 1:25 to 25:1, about 1:20 to 20:1, about 1:18 to 18:1, about 1:16 to 16:1, about 1:14 to 14:1, or about 1:12 to 1:12, also about 1:10 to 10:1, such as about 1:9 to 9:1, about 1:8 to 8:1, about 1:7 to 7:1, about 1:6 to 6:1, also from 1:5 to 5:1, such as from 1:4 to 4:1, e.g. from 1:3 to 3:1, such as from 1:2 to 2:1.

For the administration to a mammal, such as a human, the chemical complex may be administered directly, eventually provided in a capsule or the like. More convenient, the complex may be formulated into a composition comprising the chemical complex and optionally, one or more acceptable excipients. Alternatively, the combination of the two agents may also be formulated into a composition without being provided as a chemical complex.

Thus, an important aspect of the present invention relates to a composition comprising:
i) a beta-2 adrenoceptor agonist;
ii) an aminosugar; and optionally
iii) one or more acceptable excipients or carriers.

It is to be understood that the "beta-2 adrenoceptor agonist" and the "aminosugar" of the composition are as defined *supra.* In one embodiment, the composition comprises the combination of beta-2 adrenoceptor agonist and the aminosugar in the form of a chemical complex as defined herein. Thus, the aminosugar may be selected from the group consisting of glucosamine, galactosamine, mannosamine, derivatives and salts thereof, e.g. wherein the aminosugar is N-acetylglucosamine, N-acetylgalactosamine or N-acetylmannosamine. A preferred composition comprises glucosamine sulfate, glucosamine hydrochloride and/or N-acetylglucosamine. Moreover, the molar ratio or mass ratio between the beta-2 adrenoceptor agonist and the aminosugar in the composition may be as defined for the complex, as discussed *supra.*

The term "composition" is intended to mean cosmetic compositions, pharmaceutical compositions, nutritional compositions such as food supplements as well as compositions in the field of cosmeceuticals and neutraceuticals.

According to the invention, the above-mentioned chemical complexes or compositions may be combined with any other therapeutically active agents in order to strengthen, improve, potentiate, or prolong the therapeutic actions of said complexes and said compositions. Thus according to the invention, the composition or complexes may further comprise one or more therapeutically active agents.

The compositions according to the present invention may be formulated for oral, topical, transdermal, or parenteral administration, preferably oral or topical administration.
In a suitable embodiment of the invention, the compositions are used for oral administration. In another suitable embodiment of the invention the compositions are used for topical administration.

The beta-2 adrenoceptor agonist and the aminosugar may together be comprised in a single formulation or may each individually be comprised in separate formulations. The separate formulations may be administered in a simultaneous or non-simultaneous manner. As stated, the beta-2 adrenoceptor agonist and the aminosugar are together comprised in a single formulation.

The active ingredients of the chemical complex or pharmaceutical composition of the present invention need not be administered as one pharmaceutical entity, but may of course be administered as individual compounds or pharmaceutical compositions.

In addition to the formulations described previously, the compositions of the invention may also be formulated as a depot preparation. Such long acting formulations may be administered by implantation (for example subcutaneously or intramuscularly) or by intramuscular injection. Thus, for example, the compositions may be formulated with suitable polymeric or hydrophobic materials (for example as an emulsion in an acceptable oil) or ion exchange resins, or as sparingly soluble derivatives, for example, as a sparingly soluble salt.

The pharmaceutical compositions for oral, topical, transdermal, or parenteral administration may be in form of, e.g., solid, semi-solid or fluid compositions and formulated according to conventional pharmaceutical practice, see, e.g., "Remington: The science and practice of pharmacy" 20^{th} ed. Mack Publishing, Easton PA, 2000 ISBN 0-912734-04-3 and "Encyclopedia of Pharmaceutical Technology", edited by Swarbrick, J. & J. C. Boylan, Marcel Dekker, Inc., New York, 1988 ISBN 0-8247-2800-9.

The choice of pharmaceutically acceptable excipients in a composition for use according to the invention and the optimum concentration thereof is determined on the basis of the selection of the beta-2 adrenoceptor agonist, selection of the aminosugar, the kind of dosage form chosen and the mode of administration. However, a person skilled in the art of pharmaceutical formulation may find guidance in e.g., "Remington: The science and practice of pharmacy" 20^{th} ed. Mack Publishing, Easton PA, 2000 ISBN 0-912734-04-3. A pharmaceutically acceptable excipient is a substance, which is substantially harmless to the individual to which the composition will be administered. Such an excipient suitably fulfils the requirements given by the national drug agencies. Official pharmacopelas such as the British Pharmacopeia, the United States of America Pharmacopeia and the European Pharmacopeia set standards for well-known pharmaceutically acceptable excipients.

For topical, trans-mucosal and trans-dermal compositions, such as administration to the mucosa or the skin, the compositions for use according to the invention may contain conventional non-toxic pharmaceutically acceptable carriers and excipients including microspheres and liposomes.

The topical, trans-mucosal and trans-dermal compositions for use according to the invention include an array of solid, semi-solid and fluid compositions. Compositions of particular relevance are e.g. pastes, ointments, hydrophilic ointments, creams, gels, hydrogels, solutions, emulsions, suspensions, lotions, liniments, resoriblets, suppositories, enema, pessaries, moulded pessaries, vaginal capsules, vaginal tablets, shampoos, jellies, soaps, sticks, sprays, powders, films, foams, pads, sponges (e.g. collagen sponges), pads, dressings (such as, e.g., absorbent wound dressings), drenches, bandages, plasters and transdermal delivery systems.

The pharmaceutically acceptable excipients for topical, trans-mucosal and trans-dermal compositions may indude solvents, buffering agents, preservatives, humectants, chelating agents, antioxidants, stabilizers, emulsifying agents, suspending agents, gel-forming agents, ointment bases, suppository bases, penetration enhancers, perfumes, skin protective agents, diluents, disintegrating agents, binding agents, lubricants and wetting agents.

The oral compositions for use according to the invention include an array of solid, semi-solid and fluid compositions. Compositions of particular relevance are e.g. solutions, suspensions, emulsions, uncoated tablets, immediate-release tablets, modified-release tablets, gastro-resistant tablets, orodispersible tablets, efferverscent tablets, chewable tablets, soft capsules, hard capsules, modified-release capsules, gastro-resistant capsules, uncoated granules, effervescent granules, granules for the preparation of liquids for oral use, coated granules, gastro-resistant granules, modified-release granules, powders for oral adminstration and powders for the preparation of liquids for oral use.

The pharmaceutically acceptable excipients may include solvents, buffering agents, preservatives, humectants, chelating agents, antioxidants, stabilizers, emulsifying agents, suspending agents, gel-forming agents, diluents, disintegratig agents, binding agents, lubricants, coating agents and wetting agents.

Typical solvents may be selected from the group comprising water, alcohols, vegetable or marine oils (e.g. edible oils like almond oil, castor oil, cacao butter, coconut oil, com oil, cottonseed oil, linseed oil, olive oil, palm oil, peanut oil, poppyseed oil, rapeseed oil, sesame oil, soybean oil, sunflower oil, and teaseed oil), mineral oils, fatty oils, liquid paraffin, polyethylene glycols, propylene glycols, glycerol, liquid polyalkylsiloxanes, and mixtures thereof.

Typical buffering agents may be selected from the group comprising of citric acid, acetic acid, tartaric acid, lactic acid, hydrogenphosphoric acid, diethylamine etc.

Typical preservatives may be selected from the group comprising parabens, such as methyl, ethyl, propyl p-hydroxybenzoate, butylparaben, isobutylparaben, isopropylparaben, potassium sorbate, sorbic acid, benzoic acid, methyl benzoate, phenoxyethanol, bronopol, bronidox, MDM hydantoin, iodopropynyl butylcarbamate, EDTA, benzalconium chloride, and benzylalcohol, or mixtures of preservatives.

Typical humectants may be selected from the group comprising glycerin, propylene glycol, sorbitol, lactic acid, urea, and mixtures thereof. Typical chelating agents are but not limited to sodium EDTA and citric acid. Typical antioxidants may be selected from the group comprising butylated hydroxy anisole (BHA), ascorbic acid and derivatives thereof, tocopherol and derivatives thereof, cysteine, and mixtures thereof. Suitable emulsifying agents may be selected from the group comprising naturally occurring gums, e.g. gum acacia or gum tragacanth; naturally occurring phosphatides, e.g. soybean lecithin; sorbitan monooleate derivatives; wool fats; wool alcohols; sorbitan esters; monoglycerides; fatty alcohols, fatty acid esters (e.g. triglycerides of fatty acids); and mixtures thereof.

Suitable suspending agents may be selected from the group comprising celluloses and cellulose derivatives such as, e.g., carboxymethyl cellulose, hydroxyethylcellulose, hydroxypropylcellulose, hydroxypropylmethylcellulose, carrageenan, acacia gum, arabic gum, tragacanth, and mixtures thereof.

Suitable gel bases and viscosity-increasing components may be selected from the group comprising liquid paraffin, polyethylene, fatty oils, colloidal silica or aluminium, zinc soaps, glycerol, propylene glycol, tragacanth, carboxyvinyl polymers, magnesium-aluminium silicates, Carbopol®, hydrophilic polymers such as, e.g. starch or cellulose derivatives such as, e.g., carboxymethylcellulose, hydroxyethylcellulose and other cellulose derivatives, water-swellable hydrocolloids, carragenans, hyaluronates (e.g. hyaluronate gel optionally containing sodium chloride), and alginates including propylene glycol alginate.

Typical ointment bases may be selected from the group comprising beeswax, paraffin, cetanol, cetyl palmitate, vegetable oils, sorbitan esters of fatty acids (Span), polyethylene glycols, and condensation products between sorbitan esters of fatty acids and ethylene oxide, e.g. polyoxyethylene sorbitan monooleate (Tween).

Typical hydrophobic ointment bases may be selected from the group comprising paraffins, vegetable oils, animal fats, synthetic glycerides, waxes, lanolin, and liquid polyalkylsiloxanes. Typical hydrophilic ointment bases are but not limited to solid macrogols (polyethylene glycols).

Suitable powder components may be selected from the group comprising alginate, collagen, lactose, powder, which is able to form a gel when applied to a wound (absorbs liquid/wound exudate).

Suitable diluents and disintegrating agents may be selected from the group comprising lactose, saccharose, emdex, calcium phosphates, calcium carbonate, calcium sulphate, mannitol, starches and microcrystaline cellulose.

Suitable binding agents may be selected from the group comprising saccharose, sorbitol, gum acacia, sodium alginate, gelatine, starches, cellulose, sodium carboxymethylcellulose, methylcellulose, hydroxypropylcellulose, polyvinylpyrrolidone and polyetyleneglycol.

Typical wetting agents may be selected from the group comprising sodium laurylsulphate and polysorbate 80.

Suitable lubricants may be selected from the group comprising talcum, magnesium stearate, calcium stearate, silicium oxide, precirol and polyethylenglycol.

Suitable coating agents may be selected from the group comprising hydroxypropylcellulose, hydroxypropylmethylcellulose, polyvinylpropylidone, ethylcellulose and polymethylacrylates.

Typical suppository bases may be selected from the group comprising oleum cacao, adeps solidus and polyethylenglycols.

The present inventor has recognised the therapeutic effect of the complexes and compositions of this invention, partly by observing the reduced inflammation of the arachidonic acid induced inflamed mouse ear upon administering the complexes and compositions. This test model is a commonly employed method for screening and evaluation of anti-inflammatory drugs.

Thus, in a broadly sense the chemical complexes or compositions provides an immunomodulating effect. Moreover, the inventor has recognised that a number of diseases or conditions with similarities in the etiology of the inflammatory reactions that are provoked in the arachidonic acid induced inflamed mouse ear may be effectively treated by the present complexes and compositions of the invention. Such diseases and conditions relate in general to those associated with hypersensitivity reactions and inflammatory reactions. In a more specific sense, the chemical complexes or compositions of the invention provides suppression of hypersensitivity reactions, suppression of inflammatory reactions, suppression of IgE mediated allergic reactions, suppression of autoimmune reactions, reduction of pain, and suppression of cancer.

Correspondingly, a further aspect of the invention relates to a method for immunomodulation in a mammal, such as a human, comprising the administration to said mammal an effective amount of a combination of a beta-2 adrenoceptor agonist and an aminosugar, or pharmaceutically acceptable salts thereof, or a chemical complex comprising a beta-2 adrenoceptor agonist and an aminosugar, or pharmaceutically acceptable salts thereof.

As used herein, the term "effective amount" relates to the effective dose to be determined by a qualified practitioner, who may titrate dosages to achieve the desired response.

Factors for consideration of dose will include potency, bloavailability, desired pharmacokinetic/pharmacodynamic profiles, condition of treatment, patient-related factors (e.g. weight, health, age, etc.), presence of co-administered medications (e.g., anticoagulants), time of administration, or other factors known to a medical practitioner.

Moreover, further aspects of the invention relates to a method for the treatment of hypersensitivity disease or inflammation comprising the administration of the above mentioned chemical complexes or compositions of the invention to a mammal, preferentially a human.

As used herein, the "term treatment" relates to treatment of symptoms or prevention the relapse of symptoms in a person diagnosed with a disease related to inflammation, hypersensitivity, cancer or pain.

According to the invention, the therapeutic action of the complexes or compositions of the invention may be relevant to diseases involving hypersensitivity reactions or inflammatory reactions. Hence, the therapeutic action of the complexes or compositions of the invention may be relevant to the treatment of conditions and diseases associated with hypersensitivity reactions, such as infections (viral, bacterial, fungal, parasitic), cold and flu, contact dermatitis, insect bites, allergic vasculitis, post-operative reactions, transplantation rejection (graft-versus-host disease), and so forth.

A further aspect of the invention relates to the use of a complex of the invention for the treatment of autoimmune disorders. Correspondingly, the invention further relates to a method for the treatment or prevention of autoimmune disorders comprising the administration of the chemical complexes or compositions of the invention to a mammal, preferentially a human. Typically, the autoimmune disorders may be autoimmune hepatitis, Primary biliary cirrhosis, Primary sclerosing cholangitis, Autoimmune hemolytic anemias, Grave's disease, Myasthenia gravis, Type 1 Diabetes Mellitus, Inflammatory myopathies, Multiple sclerosis, Hashimoto's thyreoiditis, Autoimmune adrenalitis, Crohn's Disease, Ulcerative Colitis, Glomerulonephritis, Progressive Systemic Sclerosis (Scleroderma), Sjögren's Disease, Lupus Erythematosus, Primary vasculitis, Rheumatoid Arthritis, Juvenile Arthritis, Mixed Connective Tissue Disease, Psoriasis, Pemfigus, Pemfigoid, and Dermatitis Herpetiformis.

A still further aspect of the invention relates to a method for the treatment or prevention of an IgE mediated allergic reaction or condition comprising administration of the chemical complexes or compositions of the invention to a mammal, preferably to a human. The therapeutic action may be relevant to IgE mediated allergic reactions and conditions in general such as asthma, eczema (e.g. atopic dermatitis), urticaria, allergic rhinitis, anaphylaxis.

Moreover, the chemical complex or composition of the present invention may be used in a method for the treatment or prevention of any condition associated with pain. The applicant proposes the hypothesis that the therapeutic action is related to immunomodulation, possibly to a suppressing effect on hypersensitivity reactions.

Still further, the chemical complexes or compositions of the invention may be employed for the treatment or prevention of cancer of any type and at any stage. The present inventor puts forward the hypothesis that the anticancer effect is due to a combination of immunomodulating and tumour-suppressing effects of the complexes and compositions of the invention.

A still further aspect of the invention relates to the use of a combination of a beta-2 adrenoceptor agonist and an aminosugar for the preparation of a medicament for the immunomodulation of a mammal, such as a human. The immunomodulation typically results in the suppression of hypersensitivity and suppression of inflammatory reactions. The immodulation may be associated with diseases and disorders selected from the group consisting of hypersensitivity skin disease such as atopic eczema, contact dermatitis, seborrhoeic eczema and/or psoriasis; IgE mediated allergic reactions such as asthma, allergic rhinitis or anaphylaxis; autoimmune disease such as chronic inflammatory disease, Crohn's disease, ulcerative colitis, rheumatoid arthritis, gout or osteoarthritis; pain and cancer.

Accordingly, the chemical complexes or compositions of the invention are suitable for the treatment or prevention of diseases caused by inflammation of various tissues, such as the inflammation of the prostate, in particular prostatitis.

A still further aspect of the invention relates to a process for the preparation of a complex comprising i) a beta-2 adrenoceptor agonist; and ii) an aminosugar, comprising the steps of:
i) dissolving said beta-2 adrenoceptor and said aminosugar in a volatile solvent or a mixture of volatile solvents; and
ii) removing said suitable solvent so as to obtain a moisture content of at the most 5% w/w.

In principle, a plethora of solvents and mixture of solvents can be used in the preparation of complexes according to the invention. Suitable solvents or mixture of solvents are those being substantially removed upon evaporation at room temperature, at elevated temperature, under atmospheric or reduced pressure, or upon spray drying or freeze-drying. Furthermore, solvents and mixture of solvents should be suitable for dissolving or at least partially dissolving said beta-2 adrenoceptor and said aminosugar at room temperature or optionally upon heating. In a preferred embodiment of the invention, the beta-2 adrenoceptor and said aminosugar are fully dissolved in the suitable solvent or mixture of suitable solvents. Preferably, no traces of undissolved beta-2 adrenoceptor and said aminosugar is present in the solution.

Thus, according to the invention the volatile solvent is selected from the group consisting of water, water-miscible, volatile organic solvents and mixtures thereof. Suitable water-miscible organic solvents is selected from the group consisting of methanol, ethanol, propanol, iso-propanol, butanol, iso-butanol, tert-butanol, acetone , acetic acid, acetonitrile, ethers, chloroform and dichlormethane. Further suitable solvents relates to organic solvents capable of both dissolving hydrophobic and hydrophilic substances, such as those organic solvents selected from the group consisting of dimethylsulfoxide and dimethylformamids. Moreover, any other azeotrope solvents is preferred.

As stated, the process for preparation of a complex comprises removing of solvent so as to obtain a complex that is essentially dry, in solid form and in accordance with the IUPAC definition of a chemical complex. That is to say so as to form a complex with low moisture content and/or wherein the components are loosely associated at the molecular level and mixed with each other. The moisture being residues of water and/or residues of the water miscible organic solvents. Thus, in a interesting embodiment of the invention, the moisture content is at the most 3% w/w, preferably at the most about 2% w/w, more preferably at the most about 1% w/w, even more preferably at the most about 0.5 % w/w, most preferably at the most about 0.2 % w/w.

### EXAMPLES

The following examples describe the preparation of chemical complexes of the present invention.

### General method example 1-164:

The beta-2 adrenoceptor agonist and the aminosugar derivative are dissolved in as little solvent as possible. The solvent is removed by spray drying or freeze-drying. After the solvent is removed the complex is a white to yellowish powder.
The solvent is water:ethanol in any v/v % combination.

The complex is suitable for any type of product e.g. pharmaceutical products, dietary supplements and cosmetic formulations. Non-limiting examples of such products are tablets, capsules, ointments and lotions as described above.

| Example 1 to 32: Molar ratio beta-2 adrenoceptor agonist/ aminosugar derivative 1:10000 (mol/mol). | | |
|---|---|---|
| | beta-2 adrenoceptor agonist 1 mol | Aminosugar 10000 mol |
| Example 1. | Salbutamol | Glucosamine |
| Example 2. | Bambuterol | Glucosamine HCl |
| Example 3. | bitolterol | Glucosamine sulfate |
| Example 4, | Carbuterol | Glucosamine 2 sulfate, free acid |
| Example 5. | Clenbuterol | Glucosamine 2 sulfate, Na⁺ salt |
| Example 6. | Clorprenaline | Glucosamine 2 sulfate, K⁺ salt |
| Example 7. | Dioxethedrine | N-acetylglucosamine 3,4,6 sulfate, tri Na⁺ salt |
| Example 8. | Dopexamine | Galactosamine 3,6 sulfate, K⁺ salt |
| Example 9. | Ephedrine | N-acetylgalactosamine |
| Example 10. | Epinephrine | N-acetylgalactosamine sulfate |
| Example 11. | Etafedrine | N-acetylglucosamine |
| Example 12. | Ethylnorepinephrine | Glucosamine 6 sulfate, Na⁺ salt |
| Example 13. | Fenoterol | Glucosamine 3 sulfate, Na⁺ salt |
| Example 14. | Formoterol | Galactosamine 3,6 sulfate, K⁺ salt |
| Example 15. | Hexoprenaline | N-acetylgalactosamine |
| Example 16. | Isoetarine | Glucosamine HCl |
| Example 17. | Isoproterenol | Mannosamine HCl |
| Example 18. | Mabuterol | N-acetylmannosamine |
| Example 19. | Metaproterenol | Glucosamine sulfate |
| Example 20. | Methoxyphenamine | N-acetylglucosamin |
| Example 21. | Pirbuterol | N-acetylgalactosamine |
| Example 22. | Procaterol | N-acetylgalactosamine sulfate |
| Example 23. | Protokylol | N-acetylglucosamine |
| Example 24. | Reproterol | Glucosamine 6 sulfate, Na⁺ salt |
| Example 25. | Rimiterol | Glucosamine 3 sulfate, Na⁺ salt |
| Example 26. | Ritodrine | Galactosamine 3,6 sulfate, K⁺ salt |
| Example 27. | Salbutamol | N-acetylgalactosamine |
| Example 28. | Saimetrol | Glucosamine HCl |
| Example 29. | Soterenol | Mannosamine HCl |
| Example 30. | Terbutaline | N-acetylmannosamine |
| Example 31. | Tretoquinol | Glucosamine sulfate |
| Example 32. | tulobuterol | N-acetylglucosamin |

| Example 33 to 51: Molar ratio beta-2 adrenoceptor agonist / aminosugar derivative 1:6496 (mol/mol). | | |
|---|---|---|
| | Beta-2 adrenoceptor agonist 1mol | Aminosugar 6332 mol |
| Example 33. | formoterol fumerate dihydrate | Glucosamine HCl |
| Example 34. | bambuterol HCl | Glucosamine 3 sulfate, Na⁺ salt |
| Example 35. | Bitoltrol mesylate | Galactosamine 3,6 sulfate, K⁺ salt |
| Example 36. | Clenbuterol HCl | N-acetylgalactosamine |
| Example 37. | Chlorprenaline HCl, H₂O | N-acetylglucosamine |
| Example 38. | Dopexamine 2HCl | Glucosamine sulfate |
| Example 39. | Isoetarine | Glucosamine HCl |
| Example 40. | Isoproterenol | Mannosamine HCl |
| Example 41. | Mabuterol HCl | N-acetylmannosamine |
| Example 42. | Metaproterenol | Glucosamine sulfate |
| Example 43. | Methoxyphenamine HCl | N-acetylglucosamin |
| Example 44. | Pirbuterol monoacetate | N-acetylgalactosamine |
| Example 45. | Procaterol | N-acetylgalactosamine sulfate |
| Example 46. | Protokylol | N-acetylglucosamine |
| Example 47. | Reproterol HCl | Glucosamine 6 sulfate, Na⁺ salt |
| Example 48. | Rimiterol HBr | Glucosamine 3 sulfate, Na⁺ salt |
| Example 49. | Ritodrine HCl | Galactosamine 3,6 sulfate, K⁺ salt |
| Example 50. | Salbutamol sulfate | N-acetylgalactosamine |
| Example 51. | Salmetrol | Glucosamine HCl |

| Example 52 to 73: Molar ratio beta-2 adrenoceptor agonist / aminosugar derivative 1:832 (mol/mol). | | |
|---|---|---|
| | Beta-2 adrenoceptor agonist 1mol | Aminosugar 1500 mol |
| Example 52. | Soterenol | N-acetylgalactosamine |
| Example 53. | Terbutaline | Glucosamine HCl |
| | Beta-2 adrenoceptor agonist 1mol | Aminosugar 1500 mol |
| Example 54. | Tretoquinol HCl | Glucosamine 6 sulfate, free acid |
| Example 55. | Tulobuterol | Glucosamine sulfate |
| Example 56. | Salbutamol sulfate | Glucosamine HCl |
| Example 57. | Formoterol fumerate dihydrate | Glucosamin 3 sulfate, K⁺ salt |
| Example 58. | Dopexamine | Galactosamine 3,6 sulfate, K⁺ salt |
| Example 59. | Ephedrine | N-acetylgalactosamine |
| Example 60. | Epinephrine | N-acetylgalactosamine sulfate |
| Example 61. | Etafedrine | N-acetylglucosamine |
| Example 62. | Ethylnorepinephrine | Glucosamine 6 sulfate, Na⁺ salt |
| Example 63. | Fenoterol HBr | Glucosamine 3 sulfate, Na⁺ salt |
| Example 64. | Formoterol | Galactosamine 3,6 sulfate, K⁺ salt |
| Example 65. | Isoproterenol sulfate dihydrate | Mannosamine HCl |
| Example 66. | Mabuterol | N-acetylmannosamine |
| Example 67. | Metaproterenol HCl | Glucosamine sulfate |
| Example 68. | Methoxyphenamine | N-acetylglucosamin |
| Example 69. | Salbutamol | N-acetylgalactosamine |
| Example 70. | Salmeterol | Glucosamine HCl |
| Example 71. | Soterenol | Mannosamine HCl |
| Example 72. | Terbutaline sulfate | N-acetylmannosamine |
| Example 73. | Tretoquinol | Glucosamine sulfate |

| Example 74 to 91: Molar ratio beta-2 adrenoceptor agonist/ aminosugar derivative 1:405 (mol/mol). | | |
|---|---|---|
| | Beta-2 adrenoceptor agonist 1mol | Aminosugar 405 mol |
| Example 74. | Salbutamol | N-acetylglucosamin |
| Example 75. | bitolterol | Galactosamine |
| Example 76. | Carbuterol | Glucosamine HCl |
| Example 77. | Clenbuterol HCl | Glucosamine sulfate |
| Example 78. | Clorprenaline | Galactosamine 3,6 sulfate, di Na⁺ salt |
| Example 79. | Dioxethedrine | N-acetylglucosamin HCl |
| | Beta-2 adrenoceptor agonist 1mol | Aminosugar 405 mol |
| Example 80. | Ethylnorepinephrine HCl | Glucosamine 6 sulfate, Na⁺ salt |
| Example 81. | Fenoterol | Glucosamine 3 sulfate, Na⁺ salt |
| Example 82. | Formoterol | Galactosamine 3,6 sulfate, K⁺ salt |
| Example 83. | Isoproterenol | Mannosamine HCl |
| Example 84. | Mabuterol HCl | N-acetylmannosamine |
| Example 85. | Metaproterenol HCl | Glucosamine sulfate |
| Example 86. | Methoxyphenamine | N-acetylglucosamin |
| Example 87. | Salbutamol sulfate | N-acetylgalactosamine |
| Example 88. | Salmetrol | Glucosamine HCl |
| Example 89. | Soterenol HCl | Mannosamine HCl |
| Example 90. | Terbutaline sulfate | N-acetylmannosamine |
| Example 91. | Tretoquinol | Glucosamine sulfate |

| Example 92 to 115: Molar ratio beta-2 adrenoceptor agonist/ aminosugar derivative 1:130 (mol/mol). | | |
|---|---|---|
| | Beta-2 adrenoceptor agonist 1mol | Aminosugar 130 mol |
| Example 92. | Salbutamol | Glucosamine sulfate |
| Example 93. | Clenbuterol | Galactosamine |
| Example 94. | Clorprenaline | N-acetylgalactosamine 3,6 sulfate, K⁺ salt |
| Example 95. | Dioxethedrine | Glucosamine sulfate |
| Example 96. | Dopexamine | N-acetylglucosamine HCl |
| Example 97. | Ephedrine | N-acetylglucosamine 3 sulfate, free acid |
| Example 98. | Epinephrine | Galactosamine 4 sulfate, K⁺ salt |
| Example 99. | Etafedrine | N-acetylgalactosamine 3,6 sulfate, Na⁺ salt |
| Example 100. | Ethylnorepinephrine | Glucosamine 6 sulfate, K⁺ salt |
| Example 101. | Fenoterol | Glucosamine 2,3 sulfate, di Na⁺ salt |
| Example 102. | Formoterol fumerate dihydrate | N-acetylglucosamine HCl |
| Example 103. | Hexoprenaline | Glucosamine sulfate |
| Example 104. | Salmetrol | Glucosamine HCl |
| Example 105. | Soterenol | Mannosamine HCl |
| Example 106. | Terbutaline | N-acetylmannosamine |
| Example 107. | Tretoquinol | Glucosamine sulfate |
| Example 108. | Hexoprenaline | N-acetylgalactosamine |
| Example 109. | Isoetarine | Glucosamine HCl |
| | Beta-2 adrenoceptor agonist 1mol | Aminosugar 130 mol |
| Example 110. | Isoproterenol | Mannosamine HCl |
| Example 111. | Mabuterol | N-acetylmannosamine |
| Example 112. | Metaproterenol | Glucosamine sulfate |
| Example 113. | Methoxyphenamine | N-acetylglucosamin |
| Example 114. | Pirbuterol | N-acetylgalactosamine |
| Example 115. | Procaterol | N-acetylgalactosamine sulfate |

| Example 116 to 124: Molar ratio beta-2 adrenoceptor agonist/ aminosugar derivative 1: 19 (mol/mol). | | |
|---|---|---|
| | Beta-2 adrenoceptor agonist 1mol | Aminosugar 19 mol |
| Example 116. | Salbutamol | Glucosamine sulfate |
| Example 117. | Salbutamol sulfate | Glucosamine 2 sulfate, K⁺ salt |
| Example 118. | Bitolterol | Galactosamine |
| Example 119. | Carbuterol | Glucosamine |
| Example 120. | Clenbuterol | N-acetylgalactosamine 4 sulfate, K⁺ salt |
| Example 121. | Clorprenaline | N-acetyl-glucosamine HCl |
| Example 122. | Tretoquinol | Galactosamine 2 sulfate, Na⁺ salt |
| Example 123. | Hexoprenaline | Mannosamine HCl |
| Example 124. | Isoetarine | N-acetylmannosamine |

| Example 125 to 137: Molar ratio beta-2 adrenoceptor agonist/ aminosugar derivative 1:1 (mol/mol). | | |
|---|---|---|
| | Beta-2 adrenoceptor agonist 1mol | Aminosugar 1 mol |
| Example 125. | Bambuterol HCl | Glucosamine HCl |
| Example 126. | Bitolterol mesylate | N-acetyl-glucosamine |
| Example 127. | Salbutamol | Galactosamine sulfate |
| Example 128. | Formoterol fumerate dihydrate | Glucosamine 3,4,6 sulfate, free acid |
| Example 129. | Tretoquinol HCl | N-acetylgalactosamine HCl |
| Example 130. | Hexoprenaline sulfate | N-acetylgalactosamine |
| Example 131. | Broxaterol | Glucosamine HCl |
| Example 132. | Isoproterenol | Mannosamine HCl |
| | Beta-2 adrenoceptor agonist 1mol | Aminosugar 1 mol |
| Example 133. | Mabuterol | N-acetylmannosamine |
| Example 134. | Metaproterenol sulfate | Glucosamine sulfate |
| Example 135. | Methoxyphenamine | N-acetylglucosamin |
| Example 136. | Pirbuterol 2HCl | N-acetylgalactosamine |
| Example 137. | Procaterol | N-acetylgalactosamine sulfate |

| Example 138 to 143: Molar ratio beta-2 adrenoceptor agonist/ aminosugar derivative 5:1 (mol/mol). | | |
|---|---|---|
| | Beta-2 adrenoceptor agonist 5mol | Aminosugar 1 mol |
| Example 138. | Salbutamol | Galactosamine 4 sulfate, K⁺ salt |
| Example 139. | Formoterol fumerate dihydrate | N-acetylglucosamin |
| Example 140. | Fenoterol HBr | N-acetylgalactosamine |
| Example 141. | Mabuterol | Mannosamine HCl |
| Example 142. | Methoxyphenamine HCl | N-acetylglucosamine HCl |
| Example 143. | Reproterol | Glucosamine sulfate |

| Example 144 to 148: Molar ratio beta-2 adrenoceptor agonist / aminosugar derivative 50:1 (mol/mol). | | |
|---|---|---|
| | Beta-2 adrenoceptor agonist 50mol | Aminosugar 1 mol |
| Example 144. | Dioxethedrine | Glucosamine sulfate |
| Example 145. | Dopexamine 2HCl | N-acetylglucosamine |
| Example 146. | Ephedrine HCl | Galactosamine HCl |
| Example 147. | Epinephrine | N-acetylmannosamine |
| Example 148. | Salbutamol sulfate | N-acetylglucosamin HCl |

| Example 149 to 153: Molar ratio beta-2 adrenoceptor agonist / aminosugar derivative 500:1 (mol/mol). | | |
|---|---|---|
| | Beta-2 adrenoceptor agonist 500mol | Aminosugar 1 mol |
| Example 149. | Rimiterol | Glucosamine sulfate |
| | Beta-2 adrenoceptor agonist 500mol | Aminosugar 1 mol |
| Example 150. | Bitolterol mesylate | N-acetylglucosamine |
| Example 151. | Salbutamol | Galactosamine HCl |
| Example 152. | Salmetrol xinafoate | Mannosamine |
| Example 153. | Clenbuterol HCL | N-acetylglucosamin HCl |

| Example 154 to 159: Molar ratio beta-2 adrenoceptor agonist / aminosugar derivative 1000:1 (mol/mol). | | |
|---|---|---|
| | Beta-2 adrenoceptor agonist 1000mol | Aminosugar 1 mol |
| Example 154. | Mabuterol HCl | Glucosamine sulfate |
| Example 155. | Clenbuterol | N-acetylglucosamine |
| Example 156. | Salbutamol sulfate | Galactosamine HCl |
| Example 157. | Tulobuterol HCl | N-acetylgalactosamine 3,6 sulfate, di Na⁺ salt |
| Example 158. | Ritodrine HCl | N-acetylglucosamin HCl |
| Example 159. | Protokylol | Mannosamine HCl |

| Example 160 to 164: Molar ratio beta-2 adrenoceptor agonist / aminosugar derivative 10000:1 (mol/mol). | | |
|---|---|---|
| | Beta-2 adrenoceptor agonist 10000mol | Aminosugar 1 mol |
| Example 160. | Pirbuterol 2HCl | Glucosamine sulfate |
| Example 161. | Methoxyphenamine | N-acetylglucosamine |
| Example 162. | salbutamol | Galactosamine HCl |
| Example 163. | Isoetarine | N-acetylgalactosamine 3,6 sulfate, di Na⁺ salt |
| Example 164. | Fenoterol HCl | N-acetylglucosamin HCl |

### General method example 165-176:

A quantity of the beta-2 adrenoceptor agonist and the aminosugar derivative are transferred to a hard gelatine capsule.

| Example 165 to 170: Capsule 500 mg, molar ratio beta-2 adrenoceptor agonist/ aminosugar derivative 1:1000 (mol/mol). | | |
|---|---|---|
| | Beta-2 adrenoceptor agonist 1mol | Aminosugar 1000 mol |
| Example 165. | Salbutamol 239.31g/mol 0.55mg | Glucosamin HCl 215.6g/mol 499.45mg |
| Example 166. | Salbutamol sulfate 576.7g/mol 1.3mg | N-acetylglucosamine 221.2g/mol. 498.7mg |
| Example 167. | Formoterol fumerate dihydrate 840.91g/mol 0.7mg | Glucosamine sulfate 605.1g/mol 499.3mg |
| Example 168. | Formoterol 344.41g/mol 0.8mg | Galactosamine HCl 215.6g/mol 499.2mg |
| Example 169. | Fenoterol 303.36g/mol 0.7mg | Mannosamine HCl 215.6g/mol 499.3mg |
| Example 170. | Mabuterol 310.75g/mol 0.7mg | N-acetylmannosamine 221.2g/mol 499.3mg |

| Example 171 to 176: Capsule 750 mg, molar ratio beta-2 adrenoceptor agonist/ aminosugar derivative 1:53(mol/mol). | | |
|---|---|---|
| | Beta-2 adrenoceptor agonist 1mol | Aminosugar 53 mol |
| Example 171. | Dopexamine 356.51g/mol 22.7mg | Glucosamin HCl 215.6g/mol 727.3mg |
| Example 172. | Salbutamol sulfate 576.7g/mol 35.16mg | N-acetylglucosamine 221.2g/mol 714.84mg |
| Example 173. | Formoterol fumerate dihydrate 840.91g/mol 19.16mg | Glucosamine sulfate 605.1g/mol 730.84mg |
| Example 174. | Salbutamol 239.31g/mol 15.0mg | N-acetylglucosamine 221.2g/mol 735.0mg |
| Example 175. | Ephedrine 165.24g/mol 10.4mg | N-acetylmannosamine 221.2g/mol 739.6mg |
| | Beta-2 adrenoceptor agonist 1mol | Aminosugar 53 mol |
| Example 176. | Formoterol 344.41g/mol 21.94mg | Glucosamin HCl 215.6g/mol 728.06mg |

### Example 177

### Objective

The objective of this study is to assess the effect of three doses of two chemical complexes of the invention systemically administered in the arachidonic acid induced ear inflammation test in the mouse, a commonly employed method for screening and evaluation of antiinflammatory drugs. Dexamethasone was employed as reference compound.

### Test articles and vehicle

The test articles are the complexes of the invention prepared according to example 33 and example 92 (Compound 33 and Compound 92 in the following). Compound 33, Compound 92 and dexamethasone are obtained from Astion A/S, Denmark.

### Animals

The study was performed in female BALB/ca mice from M & B A/S, DK-8680 Ry. At start of the acclimatisation period the mice were in the weight range of 20 g (+/- 5g).

### Housing

The study took place in an animal room provided with filtered air. The temperature in the room was set at 21 - 23°C and the relative humidity to ≥30%. The room was illuminated to give a cycle of 12 hours light and 12 hours darkness. Light was on from 06.00 till 18.00 h.

The animals were housed in Macrolon type III cages (40x25x14 cm), 10 in each cage. The cages were cleaned and the bedding changed at least once a week.

### Bedding

The bedding was sawdust (Tapvei 4HV) from Tapvel Oy, 73620 Kortteinen, Finland.

### Diet

A complete pelleted rodent diet "Altromin 1324" from Chr. Petersen, DK- 4100 Ringsted, was available ad libitum.

### Drinking water

The animals had free access to bottles with domestic quality drinking water. The drinking water was changed daily.

### Animal randomisation and allocation

On the day of arrival the animals were randomly allocated to groups of 8 mice.

### Body weight

The animals were weighed on the day of dosing.

### Procedure

The test substances and reference compound were administered intraperitoneally in volumes of 20 ml per kg body weight 30 minutes before application of arachidonic acid to the ear.

All groups were treated with 20 µl arachidonic acid, 100 mg/ml in acetone, on the right ear.

The doses were as follows:

| Drug | Dose, mg/kg |
|---|---|
| Vehicle, PBS | -, i.p. |
| Compound 92 | 1000 mg/kg, i.p. |
| Compound 92 | 300 mg/kg, i.p. |
| Compound 92 | 100 mg/kg, i.p. |
| Compound 33 | 1000 mg/kg, i.p. |
| Compound 33 | 300 mg/kg, i.p. |
| Compound 33 | 100 mg/kg, i.p. |
| Dexamethasone | 6 mg/kg, i.p. |
| Dexamethasone | 2 mg/kg, i.p. |

One hour after the arachidonic acid application the mice were sacrificed, the ears cut from the tip with a punch biopsy knife (8 mm diameter) and weighed.

Mean weights and standard deviations were calculated. Relative ear oedema was assessed as the weight difference between right and left ear of each mouse expressed as percent of the left ear. Percent inhibition of the relative ear oedema compared with the vehicle treated groups was calculated for the test substance and reference compound treated groups.

### Clinical signs

All visible signs of ill health and any behavioural changes were recorded daily during the study. Any deviation from normal was recorded with respect to time of onset, duration and intensity.

### Statistics

Differences in relative ear oedema between the vehicle treated groups and the test substance and reference compound treated groups were tested for significance employing a non-parametric statistical method of analysis, the Mann-Whitney U test. The required level of significance was p<0.05.
All statistical analysis was performed employing the statistical software package Analyse-it v. 1.62.

### RESULTS

### Clinical signs

Arachidonic acid caused an inflammation in the right ears, which was visible after about 30 minutes. It could clearly be observed that the right ears were bright red and the left ears pale. The test articles to some extent prevented the reaction in the right ear. No test substance related adverse reactions were observed.

### Ear oedema

The various concentrations of the test artides inhibited the relative oedema as shown in the table below:

| Drug | Dose, mg per application | % Inhibition of relative ear oedema | Mann-Whitney U test |
|---|---|---|---|
| Vehicle, PBS | -, i.p. | - | - |
| Compound 92 | 1000 mg/kg, i.p. | 65 | p<0.0001 |
| Compound 92 | 300 mg/kg, i.p. | 44 | p=0.0009 |
| Compound 92 | 100 mg/kg, i.p. | 14 | p=0.0652 |
| Compound 33 | 1000 mg/kg, i.p. | 79 | p=0.0002 |
| Compound 33 | 300 mg/kg, i.p. | 64 | p<0.0001 |
| Compound 33 | 100 mg/kg, i.p. | 47 | p=0.0052 |
| Dexamethasone | 6 mg/kg, i.p. | 0 | p=0.8359 |
| Dexamethasone | 2 mg/kg, i.p. | 0 | p=0.6008 |

Compound 92 and Compound 33 yielded a dose dependent and at all doses statistically significant inhibition of ear oedema. Dexamethasone, the reference compound, surprisingly did not Inhibit ear oedema. This is attributed to a slower onset of action. Thus, the data imply that Compound 92 and Compound 33 have a faster onset of action than dexamethasone.

### CONCLUSION

The data imply that systemically administered Compound 92 and Compound 33 are potent inhibitors of arachidonic acid induced ear oedema, with a faster onset of action than dexamethasone.

### Example 178

### Objective

The objective of this study is to assess the effect of a dose of a complex according to compared to the effect of the corresponding doses of the components of the complex. All compounds were systemically administered in the arachidonic acid induced ear inflammation test in the mouse, a commonly employed method for screening and evaluation of antiinflammatory drugs. Methylprednisolone was employed as reference compound.

### Test articles and vehicle

The test articles are the complex of the invention prepared according to example 92 (Compound 92 in the following) and its components salbutamol and glucosamine sulfate. The substances were obtained from Astion A/S, Denmark.

### Animals

The study was performed in female BALB/ca mice from M & B A/S, DK-8680 Ry. At start of the acclimatisation period the mice were in the weight range of 20 g (+/- 5g).

### Housing

The study took place in an animal room provided with filtered air. The temperature in the room was set at 21 - 23°C and the relative humidity to ≥30%. The room was illuminated to give a cycle of 12 hours light and 12 hours darkness. Light was on from 06.00 till 18.00 h.

The animals were housed in Macrolon type III cages (40x25x14 cm), 10 in each cage. The cages were cleaned and the bedding changed at least once a week.

### Bedding

The bedding was sawdust (Tapvei 4HV) from Tapvei Oy, 73620 Kortteinen, Finland.

### Diet

A complete pelleted rodent diet "Altromin 1324" from Chr. Petersen, DK- 4100 Ringsted, was available ad libitum.

### Drinking water

The animals had free access to bottles with domestic quality drinking water. The drinking water was changed daily.

### Animal randomisation and allocation

On the day of arrival the animals were randomly allocated to groups of 10 mice.

### Body weight

The animals were weighed on the day of dosing and termination of the study.

### Procedure

The test substances and reference compound were administered intraperitoneally in volumes of 20 ml per kg body weight 30 minutes before application of arachidonic acid to the ear.

All groups were treated with 20 µl arachidonic acid, 100 mg/ml in acetone, on the right ear.

The doses were as follows:

| Drug | Dose, mg/kg |
|---|---|
| Vehicle, PBS | -, i.p. |
| Compound 92 | 1000 mg/kg, i.p. |
| Glucosamine sulfate | 997 mg/kg, i.p. |
| Salbutamol | 3.0 mg/kg, i.p. |
| Methylprednisolone | 30 mg/kg, i.p. |

One hour after the arachidonic acid application the mice were sacrificed, the ears cut from the tip with a punch biopsy knife (8 mm diameter) and weighed.

Mean weights and standard deviations were calculated. Relative ear oedema was assessed as the weight difference between right and left ear of each mouse expressed as percent of the left ear. Percent inhibition of the relative ear oedema compared with the vehicle treated groups was calculated for the test substance and reference compound treated groups.

### Clinical signs

All visible signs of ill health and any behavioural changes were recorded daily during the study. Any deviation from normal was recorded with respect to time of onset, duration and intensity.

### Statistics

Differences in relative ear oedema between the vehicle treated group and the other groups were tested for significance employing a non-parametric statistical method of analysis, the Mann-Whitney U test. The required level of significance will be p<0.05.
Similarly, the difference between the compound 92 treated group and the groups treated with the corresponding amounts of salbutamol and glucosamine sulfate respectively, were tested for significance to establish whether Compound 92 displays a significantly better effect than its components at the dose they occur in Compound 92. All statistical analysis was performed employing the statistical software package Analyse-it v. 1.62.

### RESULTS

### Clinical signs

Arachidonic acid caused an inflammation in the right ears, which was visible after about 30 minutes. It could clearly be observed that the right ears were bright red and the left ears pale. The test articles to some extent prevented the reaction in the right ear. No test substance related adverse reactions were observed.

### Ear oedema

The various concentrations of the test articles inhibited the relative oedema as shown in the table below:

| Drug | Dose, mg/kg | % Inhibition of relative ear oedema | Mann-Whitney U test |
|---|---|---|---|
| Vehicle, PBS | - | - | - |
| Compound 92 | 1000 mg/kg | 73 | p<0.0001 |
| Glucosamine sulfate | 997 mg/kg | 9 | p=0.1399 |
| Salbutamol | 3.0 mg/kg | 55 | p<0.0001 |
| Methylprednisolone | 30 mg/kg | 55 | p<0.0001 |

Compound 92 yielded a statistically significant inhibition of ear oedema. Glucosamine sulfate inhibited ear oedema mildly, and not statistically significantly, while Salbutamol inhibited ear oedema significantly. In the group receiving Compound 92 the relative ear oedema was 71% and 40% lower than in the groups receiving the corresponding doses of glucosamine sulfate and salbutamol, respectively. These differences were statistically significant, p<0.0001 and p=0.0076, respectively, and since Compound 92 reached a higher level of inhibition than the sum of inhibition of the corresponding doses of glucosamine sulfate and salbutamol, the data imply a synergistic effect.
Compound 92 yielded a 41% lower ear oedema than methylprednisolone and this difference was significant (p=0.0021).

### CONCLUSION

The data imply that systemically administered Compound 92 is a potent inhibitor of arachidonic acid induced ear oedema and that the surprisingly strong inhibition is obtained through a synergistic effect between the components of the complex.

### Example 179

A woman (70 years old) had been suffering from significant muscular pain for 6 years and had for periods been under treatment with different analgesics including ibuprofen and celecoxib with limited success. The last year she had continuously been taking a supplement of glucosamine sulfate, 1500 mg a day, but only obtained a small improvement of her symptoms. She was then treated with the complex of the invention disclosed in example 56 (1500 mg/day) instead of glucosamine sulfate. After two days she could feel a significant improvement compared to taking the aminosugar alone. After two weeks she was symptom free for the first time in 6 years, which persisted for another 6 weeks of treatment, where after the treatment was terminated. No adverse effects were observed.

### Example 180

A male, 68 years had been suffering from osteoarthritis of the knees for 8 years and had for periods been under treatment with different analgesics induding diclofenac codeine and rofecoxib with limited success. He had also tried the recommended dose of glucosamine in different formulations, but with very limited effect. He was then treated with the complex of the invention disclosed in example 56 (1500 mg/day). After four days he experienced a significant improvement of his major symptom pain in the knees in relation to walking. The improvement continued and after two weeks he was completely symptom free. The improvement persisted for the entire treatment period of 10 weeks, where after the treatment was terminated. No adverse effects were observed.

## Claims

1. A chemical complex comprising:
i) a beta-2 adrenoceptor agonist; and
ii) an aminosugar.

2. A chemical complex according to claim 1, wherein the beta-2 adrenoceptor agonist is selected from the group consisting of bambuterol, bitolterol, broxaterol, carbuterol, clenbuterol, clorprenaline, dioxethedrine, dopexamine, ephedrine, epinephrine, etafedrine, ethylnorepinephrine, fenoterol, formoterol, hexoprenaline, isoetarine, isoproterenol, mabuterol, metaproterenol, methoxyphenamine orciprenaline, pirbuterol, procaterol, protokylol, reproterol, rimiterol, ritodrine, salbutamol (albuterol), salmeterol, soterenol, terbutaline, tretoquinol, tulobuterol, derivatives and salts thereof.

3. The chemical complex according to any one of claims 1 or 2, wherein said aminosugar is an aminosugar derivative selected from the group consisting of an aminosugar derivative of a monosaccharide, aminosugar derivative of a di or oligosaccharide and salts thereof.

4. The chemical complex according to any one of claims 1 or 2, wherein said aminosugar is an aminosugar derivative of a monosaccharide selected from the group consisting of glucosamine, galactosamine, mannosamine, derivatives and salts thereof.

5. The chemical complex according to any one of claims 2 or 3, wherein said aminosugar derivative comprises a saccharide selected from the group consisting of glucosamine, galactosamine and mannosamine.

6. A composition comprising:
i) a beta-2 adrenoceptor agonist;
ii) an aminosugar; and optionally
iii) one or more acceptable excipients or carriers.

7. The composition according to claim 6, wherein the beta-2 adrenoceptor agonist is selected from the group consisting of bambuterol, bitolterol, carbuterol, clenbuterol, clorprenaline, dioxethedrine, dopexamine, ephedrine, epinephrine, etafedrine, ethylnorepinephrine, fenoterol, formoterol, hexoprenaline, isoetarine, isoproterenol, mabuterol, metaproterenol, methoxyphenamine, pirbuterol, procaterol, protokylol, reproterol, rimiterol, ritodrine, salbutamol (albuterol), salmeterol, soterenol, terbutaline, tretoquinol, tulobuterol, derivatives and salts thereof.

8. The composition according to any one of claims 6 or 7, wherein said aminosugar is an aminosugar derivative selected from the group consisting of an aminosugar derivative of a monosaccharide, aminosugar derivative of a di or oligosaccharide and salts thereof.

9. The composition according to any one of claims 6 or 7, wherein said aminosugar is an aminosugar derivative of a monosaccharide selected from the group consisting of glucosamine, galactosamine, mannosamine, derivatives and salts thereof.

10. The composition according to any one of claims 7 or 8, wherein said aminosugar derivative comprises a saccharide selected from the group consisting of glucosamine, galactosamine and mannosamine.

11. Use of a combination of a beta-2 adrenoceptor agonist and an aminosugar for the preparation of a medicament for the immunomodulation of a mammal, such as a human.

12. The use according to claim 11, wherein the immunomodulation is treatment of hypersensitivity and/or inflammatory reactions.

13. The use according to claim 12, wherein the immodulation is associated with diseases and disorders selected from the group consisting of hypersensitivity skin disease, atopic eczema, contact dermatitis, seborrhoeic eczema, psoriasis, IgE mediated allergic reactions, asthma, allergic rhinitis, anaphylaxis, autoimmune disease, chronic inflammatory disease, Crohn's disease, ulcerative colitis, rheumatoid arthritis, gout, osteoarthritis, pain and cancer.

14. The use according to any one of claims 11 to 13, wherein the medicament comprises a composition as defined by any one of claims 6 to 10.

15. The use according to any one of claims 11 to 13, wherein the medicament comprises a chemical complex as defined in any one of claims 1 to 5.
